# EUROPEAN PATENT APPLICATION

(11) **EP 1 405 641 A2**
(43) Date of publication of application: **07.04.2004**
(21) Application number: 03017838.8
(22) Date of filing: 05.08.2003
(51) Int. Cl.: A61K 35/74

(54) **Pharmaceutical and/or nutraceutical composition comprising a combination of probiotic non sporogenic microorganisms and spores of Bacillus subtilis and/or Bacillus clausii**

(30) Priority: 07.08.2002 IT MI20021800
(71) Applicant: Proge Farm S.r.l., 28100 Novara (IT)
(72) Inventor: Dondi, Giancarla, 28065 Cerano (NO) (IT)
(74) Representative: Valentini, Giuliano

(57) **Abstract**

The present invention relates to a combination for pharmaceutical, dietary and/or nutraceutical use comprising one or more non sporogenic probiotic microorganisms and spores of *Bacillus subtilis* and/or *Bacillus clausii,* the present invention also relates to the use of said combination in the treatment of alterations of intestinal microflora and the compositions and food products containing it.

## Description

The present invention relates to a new combination for pharmaceutical and/or nutraceutical use comprising one or more non sporogenic probiotic microorganisms and spores of *Bacillus subtilis* and/or *Bacillus clausii,* the invention also relates to the use of said combination in the treatment of alterations of intestinal microflora and the compositions containing it.

There are several products based on probiotic microorganisms that are available in commerce. Actually widely used are lactobacilli, streptococci, bifids, spores of bacilli, and so on, in various mixtures and various dosages, either in the pharmaceutical field and in the dietary and nutritional field, which are recently indicated also with the designation of "nutraceutical".

The origins of the various strains of probiotic organisms used are highly varied, in consideration of the ubiquitous presence of these microorganisms, and their effectiveness is now widely demonstrated by specific bibliography.

There has also been proposed a combination of spores of *Bacillus* coagulans (which is a bacillus usually non-resident in the intestine and erroneously classified as *Lactobacillus* sporogenes) with probiotic agents, especially with lactobacilli of the types bulgaricus and *acidophilus* and/or *Bifidobacterium bifidum* and/or *Streptococcus thermophilus* and/or with Group B vitamins (which are for example on the market in Italy under the tradename of NeoLactoflorene).

No scientific publication is available that would support the probiotic value of this type of combination, even less to support *Bacillus* coagulans per se.

A wide body of literature, on the contrary, supports the activity of the spores of *Bacillus subtilis* and/or *clausii* in reconstituting the intestinal flora. The action mechanism of such activity appears to be the immune stimulation, activated by the spores themselves, in the course of their transit through the intestine.

The wide diffusion of the product "Enterogermina®" containing them is a further demonstration of their being trusted by physicians and consumers.

Abundant literature actually demonstrates, on the contrary, that certain Lactobacilli have the capacity of dwelling in and colonizing the intestine with benefice for the host.

There has now been discovered that the combination of spores of *Bacillus subtilis* and/or *clausii* with non sporogenic probiotic microorganisms has a beneficial effect onto the intestine, said effect going far beyond the simple additive effect consisting of the sum of the activities of the individual components of the mixture.

Indeed it has surprisingly been found that the effects of such a composition onto the restoration of the altered intestinal flora, for example as a consequence of an antibiotic treatment, are produced in time periods that are unexpectedly faster as compared with those shown by the microorganisms and the spores of the invention when given alone.

In particular, there has now been discovered that the spores of *Bacillus subtilis* and/or *clausii* unfold a positive stimulus activity on strains of non sporogenic probiotic microorganisms when they are administered simultaneously therewith, thereby favouring the settlement of said non sporogenic probiotic microorganisms into the intestinal site, as well as their survival, thereby resulting in facilitating the re-colonization of the intestinal bacterial flora.

Thus, according to one of its aspects, the present invention relates to a combination for pharmaceutical and/or nutraceutical use comprising spores of *Bacillus subtilis* and/or *Bacillus clausii* and non sporogenic probiotic microorganisms.

According to the present invention, the expression "non sporogenic probiotic microorganisms" designates the strains of microorganisms which, after having been ingested generally by the oral route, provide a beneficial action onto the intestinal mucosa, said action causing the physiological microbial flora to be re-equilibrated.

According to a preferred embodiment of the present invention, the non sporogenic probiotic microorganisms are selected from the group consisting of probiotic lactobacilli, streptococci and bifids.

According to another preferred embodiment of the present invention, the non sporogenic probiotic microorganisms are lactobacilli that are selected with the method described in patent IT1289984 of the same Applicant.

According to another preferred embodiment of the present invention, the non sporogenic probiotic microorganisms are lactobacilli that are selected with the method described in the patent application n° MI99A000858 of the same Applicant.

According to a further preferred embodiment of the present invention, the non sporogenic probiotic microorganisms are selected from the group consisting of, but not limited to, *Lactobacillus salivarius, Lactobacillus paracasei, Lactobacillus rhamnosus, Lactobacillus gasseri, Lactobacillus acidophilus, Lactobacillus delbrueckii* and *Streptococcus thermophilus,* and their mixtures.

According to a further preferred embodiment of the present invention, the non sporogenic probiotic microorganisms are selected from the group consisting of *Lactobacillus acidophilus* P18806 strain, *Lactobacillus delbrueckii* P18805 strain, *Streptococcus thermophilus* P18807 strain and their mixtures, said strains being contained in the product that is marketed under the trademark Endolac®.

According to a further preferred embodiment of the present invention, the non sporogenic probiotic microorganisms are selected from the group consisting of *Lactobacillus paracasei* I 1687 strain, *Lactobacillus paracasei* I 1688 strain, *Lactobacillus salivarius* I 1794 strain and their mixtures, said strains being contained in the product that is marketed under the trademark Enterobacilli®.

According to a further preferred embodiment of the present invention, the non sporogenic probiotic microorganisms consist of *Lactobacillus "GG"* ATCC 53103, possibly in admixture with other non sporogenic probiotic microorganisms.

According to a further preferred embodiment of the present invention, the non sporogenic probiotic microorganisms consist of *Bifidobacterium bifidum, Lactobacillus rhamnosus,* possibly in admixture with other non sporogenic probiotic microorganisms.

The combination of the present invention can contain spores of *Bacillus subtilis* and/or *clausii* and/or one ore more of the above-mentioned probiotic microorganisms.

According to an especially advantageous aspect, among the *Bacillus subtilis* and/or *clausii* spores particularly preferred are the spores that are contained in the product marketed under the trademark Enterogermina®.

The ratio of the *Bacillus subtilis* and/or *clausii* spores and the non sporogenic probiotic microorganism(s) in the combination can be varied within wide ranges.

For example, the combination can comprise a ratio of the spores to said one or more non sporogenic probiotic microorganisms of about 10³/10¹¹ or 10¹¹/10³ (spores/CFU).

For the administration of the combination according to the present invention, pharmaceutical and/or nutraceutical compositions are employed, preferably in dosage unit forms.

Alternatively, the combination according to the present invention can be added into food or drinks.

Solid or liquid form food comprising the combination according to the present invention form a further subject matter of the invention

According to another of its aspects, the pharmaceutical and/or nutraceutical compositions (hereinafter referred to simply as "compositions") comprising the combination of the present invention are another subject matter of the invention.

According to a preferred embodiment of the present invention, the compositions of the invention comprise the spores and the non sporogenic probiotic microorganisms of the combination in a lyophilized form.

The combination of the present invention shows no toxicity and therefore can be administered even in great amount without causing any damage or adverse side effect.

The compositions of the invention in dosage unit form can comprise a high amount of the active ingredient, for example from 10³ to 10¹¹ spores of *Bacillus subtilis* and/or *clausii,* and from 10³ to 10¹¹ CFU of one or more non sporogenic probiotic microorganisms. Higher dosages can however be provided, if necessary or desired.

Advantageously, the compositions of the invention comprise the combination of the present invention in admixture with at least one non toxic carrier and/or excipient, preferably selected from the carriers and excipients that are commonly employed in pharmaceutical field.

The compositions of the invention are administered one or more times a day according to the needs, the health condition of the patient, and the weight and age thereof.

According to a preferred aspect, the compositions of the invention are administered orally.

For their administration, the compositions of the invention are preferably formulated as a freeze-dried product and in suitable dosage forms, for example capsules, sachets, vials, and the like.

The compositions of the invention can be prepared according to known techniques, for example by mixing the previously freeze-dried individual spore and non sporogenic probiotic microorganisms strains, possibly adding proper carriers and/or excipients.

As the proper carriers and/or excipients according to the present invention, there can be mentioned for instance lactose, maltodextrins sucrose, silica.

The compositions of the invention can contain, in addition to the combination of the present invention and the possible proper carriers and/or excipients, also other components or active ingredients which promote the growth of the microorganisms of the combination, for example prebiotic agents such as inuline, fructooligosaccharides, or vitamins as well, or compounds that are useful for the final indication of the composition.

Illustrative, non limiting examples of compositions of the invention are provided in the experimental part of the present description.

The combination of the present invention has been tested in clinical studies aiming at checking its effectiveness in the restoration of the intestinal microflora.

In particular, a study has been carried out on volunteers who had previously been subjected to an antibiotic treatment. These volunteers were subsequently administered either the inventive combination, or the individual components, namely *Bacillus subtilis* and/or *clausii* or non sporogenic probiotic microorganisms, respectively, not mixed together. The activity was checked by means of a genetic recognition test on stool samples, that is called PCR-DGGE, said test being described in Appl. Environ. Microbiol. Vol. 66(6): 2578-2588, 2000. The results of the studies carried out have shown that the effects of the combination onto the restoration of intestinal microflora appear in significantly shorter time as compared with the effects of the individual components not mixed together, thereby indicating an evident synergic action of the elements of the combination.

According to another of its aspects, the invention relates to the use of a combination as described above for the preparation of a medicament having a probiotic activity, especially a medicament for the treatment of alterations of intestinal microflora.

It turns therefore out that the combination of the present invention is useful for the restoration of intestinal microflora after an antibiotic treatment, and in the treatment of the bacterial dismicrobioses as well.

According to another of its aspects, the invention relates to a method for the prevention and/or treatment of the alterations of intestinal microflora, said method comprising administering a combination or a composition according to the invention.

The examples hereinafter illustrate the invention without limiting it in any way.

### Experimental Section

### EXAMPLE 1

A pharmaceutical composition is prepared containing, as the active ingredients:
about 10⁹ CFU of *Lactobacillus rhamnosus* , spray-dried;
about 10⁸ CFU of *Streptococcus thermophilus,* spray-dried;
about 10⁸ CFU of *Lactobacillus acidophilus,* spray-dried;
about 10⁸ spores *of Bacillus subtilis,* spray-dried;
about 1 mg of Vitamin B1;
about 1 mg of Vitamin B2.

### EXAMPLE 2

A pharmaceutical composition is prepared containing, as the active ingredients:
about 10⁹ spores of *Bacillus clausii,* spray-dried;
about 10⁹ CFU of *Lactobacildus paracasei,* spray-dried;
about 10⁷ CFU of *Lactobacillus salivarius,* spray-dried;

### EXAMPLE 3

A pharmaceutical composition is prepared containing, as the active ingredients:
about 2 x 10⁹ spores of *Bacillus clausii,* spray-dried;
about 10⁷ CFU of *Lactobacillus acidophilus,* spray-dried;
about 10⁸ CFU of *Streptococcus thermophilus,* spray-dried;
about 10⁹ CFU of *Bifidobacterium bifidum ,* spray-dried.

### EXAMPLE 4

A pharmaceutical composition is prepared containing, as the active ingredients:
about 1 x 10³ spores of *Bacillus clausii,* spray-dried;
about 10¹¹ CFU of *Lactobacillus acidophilus,* spray-dried;
about 10⁷ CFU of *Streptococcus thermophilus,* spray-dried;
about 10⁴ CFU of *Lactobacillus delbreuckii,* spray-dried.

### EXAMPLE 5

A pharmaceutical composition is prepared containing, as the active ingredients:
about 10⁹ spores of *Bacillus clausii,* spray-dried;
about 10⁸ CFU of *Lactobacillus acidophilus,* spray-dried;
about 10⁷ CFU of *Lactobacillus salivarius,* spray-dried;
about 10⁸ CFU of *Lactobacillus rhamnosus* , spray-dried;
1000 mg of inuline.

### EXAMPLE 6

A pharmaceutical composition is prepared containing, as the active ingredients:
about 10⁹ spores of *Bacillus clausii,* spray-dried;
about 10⁸ CFU of *Lactobacillus acidophilus,* spray-dried;
about 10³ CFU *Lactobacillus delbreuckii,* spray-dried.

## Claims

1. A combination for pharmaceutical and/or nutraceutical use comprising spores of *Bacillus subtilis* and/or *Bacillus clausii* and non sporogenic probiotic microorganisms.

2. A combination according to claim 1, in which said non sporogenic probiotic microorganisms are selected from the group consisting of lactobacilli, streptococci and bifids.

3. A combination according to claim 1, in which said non sporogenic probiotic microorganisms are lactobacilli that are selected with the method described in patent IT1289984 or with the method described in the patent application n° MI99A000858.

4. A combination according to claim 1, in which said non sporogenic probiotic microorganisms are selected from the group consisting of *Lactobacillus acidophilus, Lactobacillus delbrueckii, Streptococcus thermophilus, Lactobacillus salivarius, Lactobacillus paracasei,* and mixtures thereof.

5. A combination according to claim 1, in which said non sporogenic probiotic microorganisms are selected from the group consisting of *Lactobacillus acidophilus* P18806 strain, *Lactobacillus delbrueckii* P18805 strain, *Streptococcus thermophilus* P18807 strain, *Lactobacillus salivarius* I 1794 strain, *Lactobacillus paracasei* I 1687 strain, *Lactobacillus paracasei* I 1688 strain, *Lactobacillus* "GG" ATCC 53103, or *Bifidobacterium bifidum,* and mixtures thereof, possibly in admixture with other non sporogenic probiotic microorganisms.

6. A combination according to claim 1, containing two or more non sporogenic probiotic microorganisms.

7. A combination according to anyone of the preceding claims, in which said spores and each of said non sporogenic probiotic microorganisms are present in a ratio of about 10³/10¹¹ or 10¹¹/10³ (spores/CFU).

8. A pharmaceutical or nutraceutical or alimentary composition comprising the combination according to one of claims 1 to 7, optionally in combination with a probiotic agent.

9. A composition according to claim 8, in which said combination is in a spray-dried form, optionally with one or more pharmaceutically acceptable excipients or carriers.

10. The use of the combination according to anyone of claims 1 to 7 for the preparation of a probiotic medicament for the treatment of alterations of intestinal microflora.
